# FASCICULE DE BREVET EUROPEEN

(11) **EP 3 507 543 B1**
(45) Date de publication et mention de la délivrance du brevet: **29.07.2020**
(21) Numéro de dépôt: 17727296.0
(22) Date de dépôt: 11.05.2017
(51) Int. Cl.: F21V 5/00, F21V 7/00, F21Y 115/10

(54) **DISPOSITIF D'ECLAIRAGE MEDICAL AVEC DES LEDS ORIENTEES PAR DES LANGUETTES PREDECOUPEES DANS UNE CARTE DE CIRCUIT IMPRIME**
MEDIZINISCHE BELEUCHTUNGSVORRICHTUNG MIT LEDS, DIE DURCH IN EINER LEITERPLATTE VORGESCHNITTENE LASCHEN AUSGERICHTET SIND
MEDICAL ILLUMINATION DEVICE WITH LEDS ORIENTED BY TABS PRE-CUT IN A PRINTED CIRCUIT BOARD

(30) Priorité: 05.09.2016 FR 1658229
(43) Date de publication de la demande: 10.07.2019
(73) Titulaire: Maquet SAS, 45160 Ardon (FR)
(72) Inventeur: SENELIER, Grégory, 45047 Cedex 2 Orleans (FR); BRETON, Jean-Philippe, 45074 Cedex 2 Orleans (FR); COMTE, Lionel, 45074 Cedex 2 Orleans (FR)
(74) Mandataire: Prugneau, Philippe
(86) Numéro de dépôt international: PCT/FR2017/051132
(87) Numéro de publication internationale: WO 2018/042086

(56) Documents cités:
- EP-A1- 2 031 295
- US-A1- 2008 238 323
- US-A1- 2012 182 731

## Description

### Domaine technique

L'invention concerne de façon générale un dispositif d'éclairage médical pour l'éclairage d'un champ opératoire comprenant plusieurs sources de lumière à LEDs coopérant avec des optiques de collimation pour faire converger la lumière sur le champ opératoire, les LEDs étant montées sur une carte de circuit imprimé plate de telle façon à présenter à travers les optiques de collimation des axes d'éclairement ayant des orientations angulaires différentes.

### Technique antérieure

De manière connue, par exemple du document US2012/0182731, un dispositif d'éclairage médical comprend plusieurs sources de lumière à LEDs montées sur une carte de circuit imprimé, les sources étant couplées à des optiques de collimation, l'ensemble étant agencé de sorte à faire converger avec des orientations angulaires différentes vers le champ opératoire des faisceaux lumineux issus des sources de lumière pour former une tache d'éclairement.

Dans ce dispositif d'éclairage médical connu, la carte de circuit imprimé plane est prise en sandwich entre un support sur lequel sont montés les optiques de collimation et une structure porteuse.

Lors du montage mécanique du dispositif d'éclairage, le support exerce une pression mécanique sur la carte de circuit imprimé de sorte à orienter, par rapport au support, des zones non flexibles de la carte de circuit imprimé portant les sources de lumière à LEDs pour faire converger les faisceaux lumineux émis par les sources de lumière.

Dans ce dispositif d'éclairage médical connu, la pression mécanique est exercée par vissage. Le réglage de l'orientation des éléments optiques sur le support et l'ajustement de la position des sources lumineuses sur la carte de circuit imprimé pour obtenir les différentes orientations angulaires sont des opérations laborieuses.

C'est pourquoi, les fabricants de dispositif d'éclairage médical cherchent à rationaliser le montage des éléments composant un dispositif d'éclairage médical en optimisant le temps de montage et les étapes de réglage.

### Exposé de l'invention

Le but de l'invention, divulguée dans la revendication 1, est de proposer un dispositif d'éclairage médical d'une autre conception permettant de palier les inconvénients énoncés ci-dessus.

Plus particulièrement, l'invention a pour objet un dispositif d'éclairage médical pour l'éclairage d'un champ opératoire comprenant plusieurs sources de lumière à LEDs coopérant avec des optiques de collimation pour faire converger la lumière sur le champ opératoire, les LEDs étant montées sur une carte de circuit imprimé plate de telle façon à présenter à travers les optiques de collimation des axes d'éclairement ayant des orientations angulaires différentes, caractérisé en ce que la carte de circuit imprimé est découpée pour former des languettes flexibles par rapport à un axe de pliage ayant chacune une extrémité libre flexible portant une LED, en ce que les optiques de collimation sont logées dans des évidements d'une structure support rigide, ces évidements s'étendant les uns par rapport aux autres suivant des directions axiales différentes qui correspondent respectivement aux différentes orientations angulaires des axes d'éclairement, en ce que chaque languette flexible portant une LED qui est associée à une optique de collimation logée dans un évidement de la structure support s'étend suivant une certaine direction axiale qui est coplanaire avec l'axe d'éclairement de l'optique de collimation et est déformée sous contrainte de pliage suivant l'axe de pliage de telle sorte que son extrémité libre flexible est orientée sensiblement perpendiculairement à l'axe d'éclairement, et en ce que chaque languette flexible est retenue, en position de déformation sous contrainte, à la structure support rigide, par vissage, collage, bouterollage ou par un cran de la structure support qui se verrouille par clipsage sur le bord de la languette.

Le dispositif d'éclairage médical selon l'invention peut encore présenter les particularités suivantes :
- chaque languette flexible peut être retenue en position de déformation sous contrainte par un cran de la structure support qui se verrouille par clipsage sur le bord de l'extrémité libre de la languette ;
- chaque languette flexible peut avoir un axe de pliage qui est globalement perpendiculaire à un axe longitudinal de la languette et qui peut être réalisé par une découpe de fragilisation ;
- les optiques de collimation peuvent être aussi maintenues par clipsage dans les évidements de la structure support ;
- chaque optique de collimation peut être pourvue d'au moins un plot de positionnement qui vient s'engager dans un trou de positionnement correspondant prévu sur l'extrémité libre de la languette correspondante ;
- la structure support est de préférence en plastique moulé ;
- il peut avoir la forme d'une coupole ;
- la coupole peut être destinée à être suspendue au plafond d'un bloc opératoire ;
- la coupole peut présenter une forme générale en croix formée par quatre modules d'éclairage ;
- la structure support peut être solidaire d'une structure porteuse formant le châssis de la coupole ;
- les languettes flexibles peuvent être obtenues par un découpage de la carte de circuit imprimé ;
- une LED peut être disposée à l'extrémité libre de chaque languette, cette extrémité libre ayant la forme d'un disque ;
- chaque évidement peut avoir une forme tronconique avec une petite base, orientée du côté de la structure porteuse de l'éclairage et une grande base, orientée du côté du champ opératoire ;

L'invention s'étend à un procédé de montage d'un dispositif d'éclairage décrit précédemment, caractérisé en ce qu'il peut comprendre les étapes suivantes :
- on insère les optiques de collimation dans les évidements de la structure support qui sont alors verrouillés en position par des crans,
- puis on vient fixer la plaque de circuit imprimé portant les LEDs sur la structure support suivant une certaine position relative,
- puis ensuite, les languettes sont pliées en direction de la structure support jusqu'à venir en butée contre les optiques de collimation et sont simultanément verrouillées en position par les crans.

Au cours du procédé on peut utiliser une vis centrale pour fixer la plaque de circuit imprimé portant les LEDs à la structure support.

Avec cet agencement selon l'invention, on peut avoir une ou plusieurs lentilles et une ou plusieurs LEDs réparties sur une ou plusieurs cartes de circuit imprimé.

La fixation relative des éléments entre eux par clipsage est simplifiée mais on peut aussi utiliser une fixation par vissage, collage, bouterollage ou analogue.

La fixation par clipsage présente l'avantage de rendre ces éléments démontables.

L'agencement selon l'invention présente l'avantage d'être simple à réaliser car le support interface pour les optiques de collimation remplit différentes fonctions, à savoir une fonction mécanique pour porter les optiques et une fonction de réglage optique pour l'orientation des axes d'éclairement des LEDs.

Pour la découpe des languettes flexibles dans la plaque de circuit imprimé, il est important de ménager pour chaque languette un axe de pliage qui est globalement perpendiculaire à l'axe longitudinal de la languette. Cet axe de pliage peut être conçu de différentes façons à la portée de l'Homme du métier, par exemple par une découpe de fragilisation.

### Présentation sommaire des dessins

La présente invention sera mieux comprise et d'autres avantages apparaîtront à la lecture de la description qui suit et des dessins annexés dans lesquels :
- la figure 1 est une représentation schématique d'un dispositif d'éclairage médical à coupole d'éclairage axiale pour l'éclairage d'un champ opératoire ;
- la figure 2 est une vue de la face avant en perspective d'un dispositif d'éclairage ;
- la figure 3 est une illustration schématique d'une partie d'une carte de circuit imprimé découpée selon l'invention ;
- la figure 4A est une vue de dessous en perspective d'une structure support pour des optiques de collimation du dispositif d'éclairage selon l'invention ;
- la figure 4B est une vue de dessus en perspective de la structure support de la figure 4A ;
- la figure 5 est une vue en coupe transversale d'une partie du dispositif d'éclairage médical selon l'invention.

### Description d'un mode de réalisation

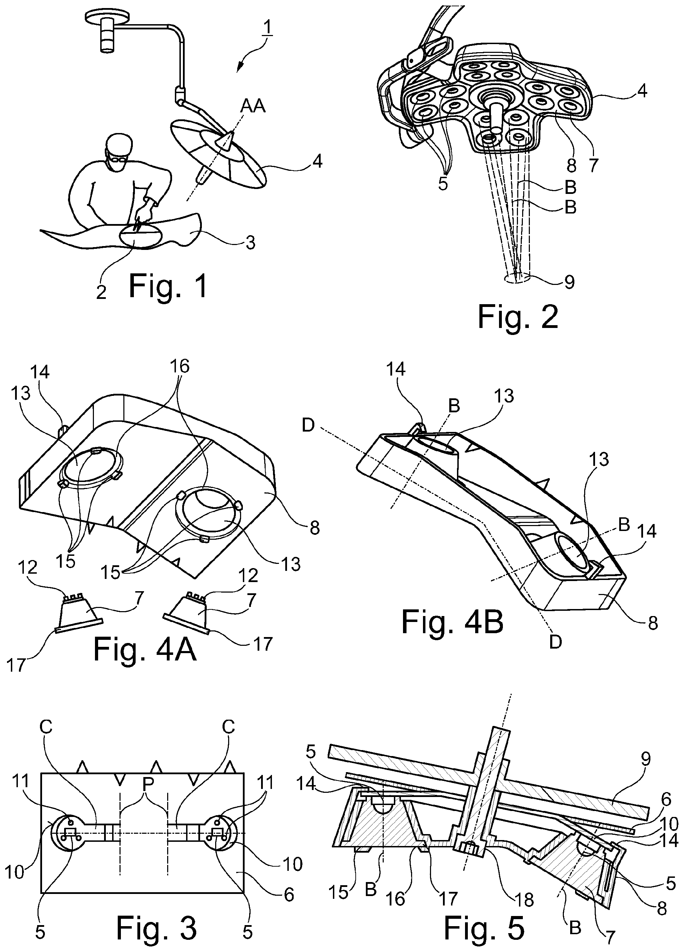

La figure 1 illustre un dispositif d'éclairage 1 médical utilisé dans un bloc opératoire pour former une tache d'éclairement 2 sur un champ opératoire 3.

Ce dispositif d'éclairage 1 a ici la forme d'une coupole 4, suspendue au plafond du bloc opératoire et qui a généralement un axe d'éclairement indiqué par la référence AA.

Sur la figure 2, on a illustré la face avant en perspective d'un dispositif d'éclairage 1 analogue à celui de la figure 1 et dont la coupole 4 présente ici une forme générale en croix formée par quatre modules d'éclairage ayant chacun quatre sources de lumière 5, ici des LEDs réparties aux coins d'un carré.

Les LEDs sont montées sur une plaque de circuit imprimé 6 qui est plate avant son montage dans le dispositif d'éclairage 1 et qui est illustrée sur la figure 3.

Sur la figure 2, on a illustré par les traits interrompus les axes d'éclairement respectifs B de quatre LEDs d'un module d'éclairage pour montrer qu'ils convergent sur le champ opératoire 3 pour former une tache d'éclairement 2 plus ou moins grande.

Ces axes d'éclairement B ont donc des orientations angulaires différentes pour converger.

Sur la figure 2, la référence 7 désigne une optique de collimation 7 ou lentille qui est montée dans une structure support 8 interface dans laquelle sont disposées plusieurs optiques de collimation 7.

La structure support 8 est solidaire d'une structure porteuse 9 formant le châssis de la coupole 4 d'éclairage comme montré sur la figure 5.

La figure 3 illustre ici une partie d'une carte de circuit imprimé 6 ou "PCB" qui est rigide et plate avant son montage dans la coupole 4.

Selon l'invention, la carte de circuit imprimé 6 est formée de languettes 10 flexibles qui s'étendent chacune suivant une certaine direction axiale indiquée par la référence C pour pouvoir être déformées par pliage.

Ces languettes 10 flexibles sont obtenues par exemple par un découpage de la carte de circuit imprimé 6.

Sur cette figure 3, on a indiqué par la référence P l'axe de pliage de chaque languette 10.

On a représenté sur la figure 3 une LED disposée à l'extrémité libre de chaque languette 10, cette extrémité libre ayant ici la forme d'un disque.

La longueur de chaque languette 10 peut être de quelques centimètres.

Chaque languette 10 flexible est ici pourvue à son extrémité libre de trois découpes ou trous de positionnement 11 disposés en triangle autour de la LED. Ces trous de positionnement 11 sont destinés à recevoir des plots de positionnement 12 correspondants qui sont prévus sur chaque optique de collimation 7.

Selon l'invention, les optiques de collimation 7 sont logées dans des évidements 13 de la structure support 8 montrés sur les figures 4A et 4B.

Sur ces figures 4A et 4B, la structure support 8 est montrée en partie avec seulement deux évidements 13.

Cette structure support 8 interface rigide est avantageusement réalisée en matière plastique moulé.

Les évidements 13 s'étendent dans la structure support 8 moulée les uns par rapport aux autres suivant des directions axiales indiquées par la référence B sur la figure 4B qui correspondent respectivement aux orientations angulaires des axes d'éclairement B sur la figure 2 et chaque languette 10 flexible portant une LED qui est associée à une optique de collimation 7 logée dans un évidement 13 de la structure support 8 interface s'étend suivant une direction longitudinale indiquée par la référence C sur la figure 3 qui est coplanaire avec l'axe d'éclairement B de sorte à pouvoir être déformée sous une contrainte de pliage suivant l'axe de pliage P de telle sorte que son extrémité libre vient se placer sensiblement perpendiculairement à l'axe d'éclairement B de l'optique de collimation 7 correspondant.

Dans l'exemple des figures 4A et 4B, chaque évidement 13 a ici une forme tronconique avec une petite base, orientée du côté de la structure porteuse 9 de l'éclairage et une grande base, orientée du côté du champ opératoire 3.

Sur une face de la structure support 8, il est prévu un cran 14 flexible visible sur la figure 4B à la périphérie de la petite base des évidements 13 qui sert à retenir en position de déformation sous contrainte (position pliée) la languette 10 comme visible sur la figure 5.

Ce cran 14 se verrouille, par exemple par clipsage, ici sur le bord de la languette 10 et plus précisément sur le bord circulaire de l'extrémité libre de la languette 10.

Sur l'autre face de la structure support 8 interface, il est prévu ici trois crans 15 flexibles visibles sur la figure 4A à la périphérie de la grande base des évidements 13 qui servent à retenir en position, par exemple par clipsage, les optiques de collimation 7 dans les logements 13.

La référence 16 sur la figure 4A désigne un épaulement annulaire 16 servant de butée de maintien pour les optiques de collimation 7 dans les évidements 13.

Les optiques de collimations 7, illustrées schématiquement sur la figure 4A ont une forme complémentaire à celle des évidements 13, avec une grande base pourvue d'une collerette circulaire 17 et une petite base pourvue des plots de positionnement 12, ici en saillie axiale par rapport à l'axe du cône et s'étendant vers l'extérieur du cône.

Pour le montage du dispositif d'éclairage 1 selon l'invention, on commence d'abord par insérer les optiques de collimation 7 dans les logements 13 de la structure support 8 interface qui sont alors verrouillés en position par les crans 15.

Puis on vient fixer la plaque de circuit imprimé 6 portant les LEDs 5 sur la structure support 8 suivant une certaine position relative par exemple à l'aide d'une vis centrale 18 qui est représentée très schématiquement sur la figure 5.

Puis ensuite, les languettes 10 sont pliées en direction de la structure support 8 jusqu'à venir en butée contre les optiques de collimation 7 et sont simultanément verrouillées en position par les crans 14 comme illustré sur la figure 5.

Enfin l'ensemble est solidarisé à la structure porteuse 9 de la coupole d'éclairage.

On comprendra que selon l'invention, il n'y a pas d'étape de réglage de la position des optiques de collimation 7, car celles-ci mises en place dans la structure support 8 interface sont déjà orientées de manière convergente dans la structure support 8 interface.

De plus, on comprendra aussi que selon l'invention, il n'est pas nécessaire de déformer toute la carte de circuit imprimé 6 pour obtenir des orientations angulaires différentes pour les sources de lumière 5 à LEDs.

## Revendications

1. Dispositif d'éclairage (1) médical pour l'éclairage d'un champ opératoire (3) comprenant plusieurs sources de lumière (5) à LEDs coopérant avec des optiques de collimation (7) pour faire converger la lumière sur ledit champ opératoire (3), lesdites LEDs étant montées sur une carte de circuit imprimé (6) plate de telle façon à présenter à travers lesdites optiques de collimation des axes d'éclairement (B) ayant des orientations angulaires différentes, ladite carte de circuit imprimé (6) étant découpée pour former des languettes (10) flexibles par rapport à un axe de pliage (P) ayant chacune une extrémité libre flexible portant une LED, **caractérisé en ce que** lesdites optiques de collimation (7) sont logées dans des évidements (13) d'une structure support (8) rigide, ces évidements (13) s'étendant les uns par rapport aux autres suivant des directions axiales différentes qui correspondent respectivement auxdites différentes orientations angulaires desdits axes d'éclairement (B), **en ce que** chaque languette (10) flexible portant une LED qui est associée à une optique de collimation (7) logée dans un évidement (13) de ladite structure support (8) s'étend suivant une certaine direction axiale (C) qui est coplanaire avec ledit axe d'éclairement (B) de ladite optique de collimation (7) et est déformée sous contrainte de pliage suivant ledit axe de pliage (P) de telle sorte que son extrémité libre flexible est orientée sensiblement perpendiculairement audit axe d'éclairement, et **en ce que** chaque languette flexible est retenue, en position de déformation sous contrainte, à ladite structure support (8) rigide, par vissage, collage, bouterollage ou par un cran (14) de ladite structure support (8) qui se verrouille par clipsage sur le bord de ladite languette (10).

2. Dispositif selon la revendication 1, **caractérisé en ce que** chaque languette (10) flexible a un axe de pliage (P) qui est globalement perpendiculaire à un axe longitudinal (C) de ladite languette et qui est réalisé par une découpe de fragilisation.

3. Dispositif d'éclairage selon la revendication 1 ou 2, **caractérisé en ce que** lesdites optiques de collimation (7) sont maintenues par clipsage dans lesdits évidements (13) de ladite structure support (8).

4. Dispositif d'éclairage selon l'une des revendications 1 à 3, **caractérisé en ce que** chaque optique de collimation (7) est pourvue d'au moins un plot de positionnement (12) qui vient s'engager dans un trou de positionnement (11) correspondant prévu sur ladite extrémité libre de ladite languette (10) correspondante.

5. Dispositif d'éclairage selon l'une des revendications 1 à 4, **caractérisé en ce que** ladite structure support (8) est en plastique moulé.

6. Dispositif d'éclairage selon l'une des revendications 1 à 5, **caractérisé en ce qu'**il a la forme d'une coupole (4) suspendue au plafond d'un bloc opératoire.

7. Dispositif d'éclairage selon la revendication 6, **caractérisé en ce que** ladite coupole (4) présente une forme générale en croix formée par quatre modules d'éclairage.

8. Dispositif d'éclairage selon la revendication 6, **caractérisé en ce que** ladite structure support (8) est solidaire d'une structure porteuse (9) formant le châssis de ladite coupole (4).

9. Dispositif d'éclairage selon la revendication 1, **caractérisé en ce que** lesdites languettes flexibles (10) sont obtenues par un découpage de ladite carte de circuit imprimé (6) ayant chacun quatre sources de lumières (5) à s réparties aux coins d'un carré.

10. Dispositif d'éclairage selon la revendication 1, **caractérisé en ce qu'**une LED (5) est disposée à l'extrémité libre de chaque languette (10), cette extrémité libre ayant la forme d'un disque.

11. Dispositif d'éclairage selon la revendication 8, **caractérisé en ce que** chaque évidement (13) a une forme tronconique avec une petite base, orientée du côté de ladite structure porteuse (9) de l'éclairage et une grande base, orientée du côté dudit champ opératoire (3).

12. Procédé de montage d'un dispositif d'éclairage selon la revendication 1, **caractérisé en ce qu'**il comprend les étapes suivantes :
- on insère lesdites optiques de collimation (7) dans lesdits évidements (13) de ladite structure support (8) qui sont alors verrouillés en position par des crans (15),
- puis on vient fixer ladite plaque de circuit imprimé (6) portant lesdites LEDs (5) sur ladite structure support (8) suivant une certaine position relative,
- puis ensuite, lesdites languettes (10) sont pliées en direction de ladite structure support (8) jusqu'à venir en butée contre lesdites optiques de collimation (7) et sont simultanément verrouillées en position par lesdits crans (14).

13. Procédé selon la revendication 12, **caractérisé en ce que** l'on utilise une vis centrale (18) pour fixer ladite plaque de circuit imprimé (6) portant lesdites LEDs (5) à ladite structure support (8).

## Patentansprüche

1. Medizinische Beleuchtungsvorrichtung (1) für die Beleuchtung eines Operationsfelds (3), umfassend mehrere Lichtquellen (5) mit LEDs, die mit Kollimieroptiken (7) zusammenwirken, um das Licht auf das Operationsfeld (3) konvergieren zu lassen, wobei die LEDs auf einer flachen, gedruckten Schaltkreisplatine (6) derart montiert sind, dass sie durch die Kollimieroptiken hindurch Beleuchtungsachsen (B) aufweisen, die unterschiedliche Winkelorientierungen aufweisen, wobei die gedruckte Schaltkreisplatine (6) ausgeschnitten ist, um Laschen (10) zu bilden, die in Bezug auf eine Faltachse (P) flexibel sind und jeweils ein freies, flexibles Ende, das eine LED trägt, aufweisen, **dadurch gekennzeichnet, dass** die Kollimieroptiken (7) in Ausnehmungen (13) einer steifen Stützstruktur (8) untergebracht sind, wobei diese Ausnehmungen (13) sich die eine in Bezug auf die anderen gemäß verschiedener axialer Richtungen erstrecken, die jeweils den vorgenannten verschiedenen Winkelorientierungen der Beleuchtungsachsen (B) entsprechen, dass jede flexible Lasche (10), die eine einer in einer Ausnehmung (13) der Stützstruktur (8) untergebrachten Kollimieroptik (7) zugeordnete LED trägt, sich gemäß einer bestimmten axialen Richtung (C), die komplanar mit der Beleuchtungsachse (B) der Kollimieroptik (7) ist und die unter Faltbeanspruchung gemäß der Faltachse (P) derart deformiert wird, dass ihr flexibles freies Ende im Wesentlichen senkrecht zu der Beleuchtungsachse ausgerichtet ist, und
dass jede flexible Lasche unter Beanspruchung in Deformationsposition an der steifen Stützstruktur (8) durch Verschraubung, Klebung, Vernietung oder durch eine Raste (14) der Stützstruktur (8), die sich durch Verklipsen auf dem Rand der Lasche (10) verriegelt, gehalten ist.

2. Vorrichtung nach Anspruch 1, **dadurch gekennzeichnet, dass** jede flexible Lasche (10) eine Faltachse (P) aufweist, die im Wesentlichen senkrecht zu einer Längsachse (C) der Lasche ist und die durch einen Schwächungszuschnitt verwirklicht ist.

3. Beleuchtungsvorrichtung nach Anspruch 1 oder 2, **dadurch gekennzeichnet, dass** die Kollimieroptiken (7) durch Verklipsen in den Ausnehmungen (13) der Stützstruktur (8) gehalten sind.

4. Beleuchtungsvorrichtung nach einem der Ansprüche 1 bis 3, **dadurch gekennzeichnet, dass** jede Kollimieroptik (7) mit wenigstens einem Positionierkontakt (12) ausgestattet ist, der in ein entsprechendes, auf dem freien Ende der entsprechenden Lasche (10) vorgesehenes Positionierloch (11) einsetzbar ist.

5. Beleuchtungsvorrichtung nach einem der Ansprüche 1 bis 4, **dadurch gekennzeichnet, dass** die Stützstruktur (8) aus gegossenem Kunststoff ist.

6. Beleuchtungsvorrichtung nach einem der Ansprüche 1 bis 5, **dadurch gekennzeichnet, dass** sie die Gestalt einer Kuppel (4) aufweist, die an der Decke eines Operationsblocks aufgehangen ist.

7. Beleuchtungsvorrichtung nach Anspruch 6, **dadurch gekennzeichnet, dass** die Kuppel (4) eine kreuzförmige Gesamtgestalt aufweist, die durch vier Beleuchtungsmodule gebildet ist.

8. Beleuchtungsvorrichtung nach Anspruch 6, **dadurch gekennzeichnet, dass** die Stützstruktur (8) mit einer Tragstruktur (9) verbunden ist, die das Chassis der Kuppel (4) bildet.

9. Beleuchtungsvorrichtung nach Anspruch 1, **dadurch gekennzeichnet, dass** die flexiblen Laschen (10) durch einen Zuschnitt der gedruckten Schaltkreisplatine (6) erhalten werden, welcher jeder vier an den Ecken eines Quadrats verteilte Lichtquellen (5) mit LEDs aufweist.

10. Beleuchtungsvorrichtung nach Anspruch 1, **dadurch gekennzeichnet, dass** eine LED (5) am freien Ende jeder Lasche (10) angeordnet ist, wobei dieses freie Ende die Gestalt einer Scheibe aufweist.

11. Beleuchtungsvorrichtung nach Anspruch 8, **dadurch gekennzeichnet, dass** jede Ausnehmung (13) eine Kegelstumpfform aufweist, mit einer in Richtung der Seite der Tragstruktur (9) der Beleuchtung orientierten kleinen Basis und einer in Richtung des Operationsfelds (3) orientierten großen Basis.

12. Verfahren zum Montieren einer Beleuchtungsvorrichtung nach Anspruch 1, **dadurch gekennzeichnet, dass** es die folgenden Schritte umfasst:
- Einsetzen der Kollimieroptiken (7) in die Ausnehmungen (13) der Stützstruktur (8), die dann durch Rasten (15) in Position verriegelt sind,
- anschließend Fixieren der die LEDs (5) tragenden gedruckten Schaltkreisplatine (6) auf der Stützstruktur (8) gemäß einer bestimmten Relativposition,
- anschließend Falten der Laschen (10) in Richtung der Stützstruktur (8), bis sie gegen die Kollimieroptiken (7) anschlagen und zugleich in Position durch die Rasten (14) verriegelt sind.

13. Verfahren nach Anspruch 12, **dadurch gekennzeichnet, dass** eine mittige Schraube (18) zum Fixieren der die LEDs (5) tragenden gedruckten Schaltkreisplatine (6) an der Stützstruktur (8) eingesetzt wird.

## Claims

1. A medical lighting device (1) for illuminating an operative field (3), which device comprises a plurality of light-emitting diode (LED) light sources (5) that cooperate with collimator optical systems (7) to cause light to converge on the operative field (3), said LEDs being mounted on a flat printed circuit board (6) in such a manner that their illumination axes (B) passing through the collimator optical systems have different angular orientations, said printed circuit board (6) being cut out to form tongues (10), each of which is flexible about a respective bending axis (P), and each of which has a flexible free end carrying an LED, said medical lighting device being **characterized in that** said collimator optical systems (7) are received in recesses (13) in a rigid support structure (8), said recesses (13) extending relative to one another in different axial directions that correspond to respective ones of said different angular orientations of said illumination axes (B), **in that** each flexible tongue (10) carrying an LED that is associated with a collimator optical system (7) received in a recess (13) in said support structure (8) extends in a certain axial direction (C) that is coplanar with said illumination axis (B) of said collimator optical system (7) and is deformed under bending stress about said bending axis (P) in such a manner that its flexible free end is oriented substantially perpendicularly to said illumination axis, and **in that** each flexible tongue is held, in the stressed deformed position, against the rigid support structure (8) by screwing, adhesive bonding, thermoplastic staking, or by a catch (14) on said support structure (8) that locks by clipping onto the edge of said tongue (10).

2. A device according to claim 1, **characterized in that** each flexible tongue (10) has a bending axis (P) that is substantially perpendicular to a longitudinal axis (C) of said tongue and that is formed by scoring.

3. A lighting device according to claim 1 or claim 2, **characterized in that** said collimator optical systems (7) are held by clipping in said recesses (13) of said support structure (8).

4. A lighting device according to any one of claims 1 to 3, **characterized in that** each collimator optical system (7) is provided with at least one positioning stud (12) that comes to be engaged in a corresponding positioning hole (11) provided in said free end of said corresponding tongue (10).

5. A lighting device according to any one of claims 1 to 4, **characterized in that** said support structure (8) is made of molded plastic.

6. A lighting device according to any one of claims 1 to 5, **characterized in that** it is in the shape of a dome (4), suspended from the ceiling of an operating theater.

7. A lighting device according to claim 6, **characterized in that** said dome (4) is in the general shape of a cross formed by four lighting modules.

8. A lighting device according to claim 6, **characterized in that** said support structure (8) is secured to a carrier structure (9) forming the frame of said dome (4).

9. A lighting device according to claim 1, **characterized in that** said flexible tongues (10) are obtained by cutting them out from said printed circuit board (6) each having four light sources (5) with LEDs distributed at the corners of a square.

10. A lighting device according to claim 1, **characterized in that** a LED (5) is placed at the free end of each tongue (10), said free end being disk-shaped.

11. A lighting device according to claim 8, **characterized in that** each recess (13) is frustoconical in shape with the small base of that shape facing towards the carrier structure (9) of the lighting and its large base facing towards the operative field (3).

12. A method of assembling a lighting device according to claim 1, said method being **characterized in that** it comprises the following steps:
• inserting said collimator optical systems (7) into said recesses (13) in said support structure (8) that are then locked in position by catches (15);
• then fastening said printed circuit board (6) carrying said LEDs (5) to said support structure (8) in a certain relative position; and
• then bending said tongues (10) towards said support structure (8) until they come into abutment against the collimator optical systems (7) and simultaneously locking them in position by means of said catches (14).

13. A method according to claim 12, **characterized in that** a central screw (18) is used for fastening said printed circuit board (6) carrying said LEDs (5) to said support structure (8).
